# EUROPÄISCHE PATENTSCHRIFT

(11) **EP 2 812 448 B1**
(45) Veröffentlichungstag und Bekanntmachung des Hinweises auf die Patenterteilung: **22.06.2016**
(21) Anmeldenummer: 13712666.0
(22) Anmeldetag: 08.02.2013
(51) Int. Cl.: C12Q 1/68

(54) **VERFAHREN ZUR ANALYSE FETALER NUKLEINSÄUREN**
METHOD FOR ANALYSING FOETAL NUCLEIC ACIDS
PROCÉDÉ D'ANALYSE D'ACIDES NUCLÉIQUES FOETAUX

(30) Priorität: 10.02.2012 AT 1782012
(43) Veröffentlichungstag der Anmeldung: 17.12.2014
(73) Patentinhaber: IVF Zentren Prof. Zech Bregenz GmbH, 6900 Bregenz (AT)
(72) Erfinder: ZECH, Nicolas, A-6900 Bregenz (AT)
(74) Vertreter: Burger, Hannes
(86) Internationale Anmeldenummer: PCT/AT2013/050033
(87) Internationale Veröffentlichungsnummer: WO 2013/116889

(56) Entgegenhaltungen:
- JOYCE C HARPER ET AL: "Preimplantation genetic diagnosis: State of the ART 2011", HUMAN GENETICS, SPRINGER, BERLIN, DE, Bd. 131, Nr. 2, 12. Juli 2011 (2011-07-12) , Seiten 175-186, XP035001234, ISSN: 1432-1203, DOI: 10.1007/S00439-011-1056-Z
- EVIAN ANNUAL REPRODUCTION (EVAR) WORKSHOP GROUP 2010 ET AL: "Contemporary genetic technologies and female reproduction", HUMAN REPRODUCTION UPDATE, OXFORD UNIVERSITY PRESS, OXFORD, GB, Bd. 17, Nr. 6, 1. November 2011 (2011-11-01), Seiten 829-847, XP002691559, ISSN: 1355-4786, DOI: 10.1093/HUMUPD/DMR033 [gefunden am 2011-09-06]
- KATZ-JAFFE M G ET AL: "Proteomic analysis of individual human embryos to identify novel biomarkers of development and viability", FERTILITY AND STERILITY, ELSEVIER SCIENCE INC, NEW YORK, NY, USA, Bd. 85, Nr. 1, 1. Januar 2006 (2006-01-01) , Seiten 101-107, XP028061729, ISSN: 0015-0282, DOI: 10.1016/J.FERTNSTERT.2005.09.011 [gefunden am 2006-01-01]
- KATZ-JAFFE M G ET AL: "Analysis of protein expression (secretome) by human and mouse preimplantation embryos", FERTILITY AND STERILITY, ELSEVIER SCIENCE INC, NEW YORK, NY, USA, Bd. 86, Nr. 3, 1. September 2006 (2006-09-01), Seiten 678-685, XP028063686, ISSN: 0015-0282, DOI: 10.1016/J.FERTNSTERT.2006.05.022 [gefunden am 2006-09-01]
- VALENTIN V. VLASSOV ET AL: "Extracellular nucleic acids", BIOESSAYS, Bd. 29, Nr. 7, 1. Januar 2007 (2007-01-01) , Seiten 654-667, XP055035366, ISSN: 0265-9247, DOI: 10.1002/bies.20604

## Beschreibung

Die Erfindung betrifft ein Verfahren zum Nachweis fetaler Nukleinsäuren aus dem Überstand des Kulturmediums fetaler Zellkulturen bzw. aus der, den Embryo umgebenden Flüssigkeit innerhalb der Eizellhülle (Zona Pellucida), aus dem Blastocoel oder aus der Blastozystenhöhle.

Zelluläre fetale DNA kann sowohl während der Präimplantationsdiagnostik (PID), die sich mit der Untersuchung des Embryos vor der Einpflanzung in den Uterus beschäftigt, als auch während der Pränataldiagnostik (PND), die sich mit der Untersuchung des Fetus vor der Geburt beschäftigt, nachgewiesen werden.

Bei der Pränataldiagnostik stehen invasive Methoden, wie Chorionzottenbiopsie, Amniozentese und Nabelschnurpunktion, zur Untersuchung der genetischen Information des sich entwickelnden Fetus und nicht-invasive Methoden, wie Ultraschalluntersuchungen, zur Verfügung. Die invasiven Verfahren erfordern die Einführung einer Nadel in den Uterus, um entweder Fruchtwasser oder Gewebe der Plazenta bzw. Blut des Fetus zu entnehmen. Hierbei kann es zu Komplikationen im Verlauf der Schwangerschaft kommen, die bis zur Fehlgeburt reichen können.

Die Möglichkeit mit nicht-invasiven Methoden genetisches Material des Fetus gewinnen und analysieren zu können, kann das Risiko einer Gesundheitsschädigung des Fetus und eines ungewollten Schwangerschaftsabbruchs eliminieren. Im Blutplasma der Mutter befindet sich neben zellulären, mütterlichen Nukleinsäuren auch frei zirkulierende DNA geringer Molekulargröße. Ein Teil dieser frei zirkulierenden DNA im Blutplasma schwangerer Frauen ist fetalen Ursprungs. Nicht-invasive, pränatale Molekulardiagnostik beruht neben Ultraschalluntersuchungen auf der Analyse dieser frei zirkulierenden fetalen DNA. Der Anteil der fetalen DNA an der DNA-Gesamtmenge im mütterlichen Blutplasma liegt bei etwa drei bis sechs Prozent und steigt mit dem Fortschreiten der Schwangerschaft an. Im ersten Trimester beträgt die Konzentration fetaler DNA durchschnittlich etwa 25 Genomequivalente (GE) pro Milliliter Plasma und erhöht sich bis auf etwa 300 GE/ml im dritten Trimester. Dabei variieren die individuellen Konzentrationen fetaler DNA im Plasma um das bis zu zehnfache. Nach der Geburt fällt der Spiegel fetaler DNA im Blutplasma rasch ab und schon nach 24 Stunden sind frei zirkulierende fetale DNA-Sequenzen im Blut der Mutter nicht mehr nachweisbar und somit vor einer weiteren Schwangerschaft wieder völlig entfernt. Als Ursprung frei zirkulierender DNA werden apoptotische Prozesse in der Plazenta vermutet, die u.a. zu einer Freisetzung degradierter genomischer DNA führen. Auf diese Weise wird kontinuierlich DNA des Fetus in die Blutbahn der Mutter entlassen. Mehrere Studien haben gezeigt, dass sich im Plasma von Schwangeren fetale und maternale DNA hinsichtlich ihrer molekularen Eigenschaften unterscheiden: fetale DNA liegt überwiegend in Form kurzer bis 300 Basenpaare (bp) großer Fragmente vor, während maternale DNA vorwiegend in höhermolekularer Form (1000 bp und mehr) zirkuliert. Diese Größenunterschiede zwischen fetaler und maternaler zellfreier DNA bleiben im Verlauf der Schwangerschaft weitgehend unverändert und ermöglichen es, über geeignete Verfahren zur DNA-Aufreinigung kurze DNA-Fragmente anzureichern und damit eine DNA-Probe zu erhalten, bei der der Anteil fetaler DNA gegenüber dem dominierenden Hintergrund an maternalen DNA-Sequenzen erhöht ist. Diese Anreicherung fetaler DNA erhöht die Aussagefähigkeit molekularer Diagnostik des Fetus für die Detektion von Sequenzveränderungen/Mutationen und insbesondere für quantitative Aussagen, wie zur Diagnose von Trisomien.

Der Nachweis fetaler DNA im mütterlichen Blutplasma ermöglicht allerdings nicht die molekulargenetische Analyse des Embryos bei einer in-vitro Fertilisation (IVF) vor dem Transfer des Embryos in den Uterus der Mutter.

Um aber die Erfolgsrate bei einer in-vitro Fertilisation zu erhöhen, kommt sowohl der morphologischen als auch der molekulargenetischen Identifikation der besten Embryonen vor dem Transfer eine immer größere Bedeutung zu, was mittels Präimplantationsdiagnostik möglich ist.

Als Präimplantationsdiagnostik (PID) werden zellbiologische und molekulargenetische Untersuchungen bezeichnet, die der Entscheidung dienen, ob ein durch in-vitro Fertilisation erzeugter Embryo in die Gebärmutter eingepflanzt werden soll oder nicht. Die PID wird hauptsächlich zur Erkennung bestimmter Erbkrankheiten und Besonderheiten der Chromosomen angewendet. Sie kann aber auch zur Erzeugung eines Babys, das als Organspender für ein erkranktes Geschwister geeignet ist, eingesetzt werden ("Retterbaby") oder zur Auswahl des Geschlechts oder bestimmter erblicher Eigenschaften des Kindes.

Um eine PID durchführen zu können, ist vorgängig eine in-vitro Fertilisation notwendig. Dabei kann das Verfahren der IVF mit PID grob in fünf Schritte unterteilt werden: 1.Hormonstimulation und Eizellgewinnung, 2.Extrakorporale Befruchtung, 3.Embryobiopsie, 4.Genetische Diagnostik, 5.Embryotransfer oder Kryokonservierung. Die Schritte drei und vier machen die PID im engeren Sinn aus.

Die Embryobiopsie, also die Abspaltung einer oder zweier Zellen von einem Embryo, erfolgt in der Regel am dritten Tag nach der Befruchtung. Der Embryo besteht zu diesem Zeitpunkt gewöhnlich aus sechs bis zehn Zellen und ist von einer Schutzhülle (Zona Pellucida) umgeben.

Gemäß einem neueren Verfahren wird der Embryo erst etwa am fünften Entwicklungstag biopsiert. In diesem Entwicklungsstadium besteht der Embryo aus einer äußeren Zellgruppe, aus der die Plazenta hervorgeht (Trophoblast), und der inneren Zellmasse, aus der sich der Embryo bzw. Fötus entwickelt (Embryoblast), und wird als Blastozyste bezeichnet. Man spricht demzufolge auch von Blastozystenbiopsie. Bei einer Blastozystenbiopsie werden in der Regel dem Trophoblasten mehrere Zellen entnommen und genetisch untersucht. Nur etwa 20-40% aller befruchteten Eizellen erreichen das Stadium der expandierten Blastozyste.

Bei einer Embryobiopsie wird zuerst mit Hilfe von Säure, Laserlicht oder auf mechanischem Weg eine Öffnung in der den Embryo umgebenden Schutzhülle geschaffen. Anschließend werden dem Embryo mittels einer Saugpipette eine oder zwei Zellen entnommen. Es gibt Hinweise, dass die Abspaltung von Zellen möglicherweise die Implantationsfähigkeit des Embryos verringert. Noch wenig geklärt ist die Frage, ob die Abspaltung darüber hinaus weitere negative Auswirkungen auf die Entwicklung des Embryos oder des Kindes haben könnte. Die PID stellt ein schwierig durchzuführendes Verfahren dar, nicht zuletzt deshalb, weil gewöhnlich höchstens zwei Zellen für den Test zur Verfügung stehen und das Verfahren nicht bis schwer wiederholt werden kann. Deshalb ist das Risiko von Fehldiagnosen nicht zu vernachlässigen. Die Wahrscheinlichkeit, dass das Testergebnis korrekt ist, liegt bei etwa 90-95%. Zur Überprüfung des Ergebnisses wird allen betroffenen Paaren empfohlen, während der Schwangerschaft zusätzlich eine PND durchzuführen. Das häufigste Problem sind falsch negative Untersuchungsergebnisse aufgrund von Kontamination mit Fremd-DNA oder aufgrund des so genannten "Allelic dropout", d.h. der Analyse nur eines statt beider Allele. Bei einem falsch negativen Untersuchungsergebnis ist der Embryo Träger des Gendefektes, obwohl die Diagnose dies nicht aussagt.

Ein weiteres Problem stellt der Mosaizismus dar, wobei unter einem Mosaik ein Embryo verstanden wird, der aus genetisch verschiedenen Zellen aufgebaut ist. So kommt es vor, dass die untersuchten Zellen ein anderes Genom aufweisen als die restlichen Zellen, was zu einer Fehldiagnose führt. Mosaizismus tritt relativ häufig auf und ist auf Fehler bei der Zellteilung zurückzuführen.

Harper et al, 2011 (Human Genetics, 175-186) beschreibt einige der bekannten Präimplantationsdiagnostikverfahren. Aufgabe der vorliegenden Erfindung ist es daher Präimplantationsdiagnostik ohne Gefährdung des Embryos aber dennoch mit hoher Aussagekraft zu ermöglichen.

Die Aufgabe der Erfindung wird jeweils eigenständig durch ein Verfahren zum Nachweis fetaler Nukleinsäuren aus dem Überstand des Kulturmediums fetaler Zellkulturen einer in-vitro Fertilisation umfassend die Schritte: a) Entnahme des zellfreien, fetale Nukleinsäure enthaltenden Überstands des in-vitro Fertilisationskulturmediums aus dem zur Kultivierung der befruchteten Eizelle verwendeten Kulturgefäßes, b) Anreicherung der im Überstand enthaltenen fetalen Nukleinsäuren und c) Durchführung einer Analyse der im zellfreien Überstand vorhandenen Nukleinsäuren sowie durch ein Verfahren zum Nachweis fetaler Nukleinsäuren aus der den Embryo umgebenden Flüssigkeit innerhalb der Zona Pellucida, dem Blastocoel oder der Blastozystenhöhle umfassend die Schritte: a) Entnahme der zellfreien, fetale Nukleinsäure enthaltenden Flüssigkeit aus der, den Embryo umgebenden Flüssigkeit innerhalb der Zona Pellucida, dem Blastocoel oder der Blastozystenhöhle der Blastula oder Blastozyste, b) Anreicherung der Nukleinsäure, die in jener den Embryo umgebenden Flüssigkeit innerhalb der Zona Pellucida enthalten ist, oder der im Blastocoel oder der Blastozystenhöhle enthaltenen fetalen Nukleinsäuren und c) Durchführung einer Analyse der im zellfreien Überstand vorhandenen Nukleinsäuren gelöst.

Die Verwendung zellfreier fetaler DNA aus dem Überstand des Kulturmediums von in-vitro Kulturen der in-vitro Fertilisation anstelle von intakten Zellen zeigt die Vorteile, dass a) die Implantationsfähigkeit des Embryos nicht verringert wird, b) das Risiko eines ungewollten Schwangerschaftsabbruchs wegfällt, c) der Embryo nicht geschädigt wird, d) die Fehlerquelle durch Mosaizismus, Mikrochimerismus, Kontamination mit Fremd-DNA sinkt, e) Fehldiagnosen vermieden werden, etc.

Vorteilhaft bei der erfindungsgemäßen Methode erweist sich, dass keine zellulären Bestandteile entnommen werden müssen. Durch die nicht-invasive Präimplantationsdiagnose können fetale chromosomale Aneuploidien, monogenetische Erkrankungen, das Geschlecht, die Blutgruppe, HLA-Typisierungen, etc. festgestellt werden.

Zudem erweist sich von Vorteil, dass durch die molekulargenetischen Methoden rasch ein Ergebnis vorliegt und somit vor dem Transfer des Embryos in den Uterus der Mutter, der üblicherweise bereits spätestens 6 Tage nach der Befruchtung stattfindet, sofern keine Kryokonservierung stattfindet, eine Entscheidung ermöglicht wird.

Die fetale Nukleinsäure kann DNA und/oder RNA sein, insbesondere genomische oder mitochondriale DNA, wobei sich von Vorteil erweist, dass zur nachfolgenden Analyse standardisierte molekularbiologische Methoden verwendet werden können.

Von Vorteil ist auch, dass der Zellkulturüberstand mono und/oder sequentieller Kulturmedien analysiert werden kann, wodurch eine Analyse im Zuge der Präimplantationsdiagnostik wiederholt werden kann, sofern ein zweifelhaftes Ergebnis vorliegt, weil jederzeit wiederum fetale Nukleinsäuren aus der Zellkultur nicht-invasiv isoliert und analysiert werden können.

Der zellfreie, die Nukleinsäure enthaltende Überstand kann am Tag 1 bis 6 der in-vitro Kultur analysiert wird, wodurch bereits sehr früh während der in-vitro Fertilisation ein Ergebnis der Analyse vorliegt. So kann beispielsweise zukünftigen Eltern bereits vor der Implantation ohne Gefährdung des Embryos eine Entscheidungsgrundlage für die weitere Vorgehensweise gegeben werden. Vorzugsweise wird die zellfreie fetale DNA/RNA am Tag 3 bis 5 der in-vitro Kultur analysiert, weil zu diesem Zeitpunkt bereits ausreichend fetale DNA/RNA des Embryos im Zellkulturüberstand der in-vitro Zellkultur vorliegt um gesichert ein Ergebnis der Analyse zu erhalten und zudem das Ergebnis noch rechtzeitig vor dem Transfer des Embryos in den Uterus der Mutter vorliegt.

Vorzugsweise wird der Zellkulturüberstand aus einem die befruchtete Eizelle umgebenden Bereich vom zumindest 2-fachen Durchmesser der kultivierten befruchteten Eizelle entnommen, weil die Konzentration fetaler DNA/RNA in diesem Bereich am höchsten ist und somit wiederum die Wahrscheinlichkeit ein Ergebnis der Analyse zu erhalten am höchsten ist.

Auch ist es möglich, die Zona Pellucida des Embryos bzw. der befruchteten Eizelle an den Kulturtagen 1 bis 6 Tage der Zellkultur vor Entnahme des Zellkulturüberstandes mechanisch, chemisch oder per Laser zu öffnen, um die Konzentration von fetaler DNA/RNA zu erhöhen, da die Zona Pellucida eine natürliches Hindernis für das Austreten von DNA/RNA in das Kulturmedium darstellt.

Je nach Verfahren (ohne oder nach Eröffnen der Zona Pellucida) werden zumindest 2 µl des Zellkulturüberstands analysiert, weil dadurch ausreichend fetale DNA/RNA der Analyse zugeführt werden kann.

Die Isolierung, insbesondere Anreicherung, der fetalen Nukleinsäuren wird durch ein Verfahren ausgewählt aus einer Gruppe umfassend Zentrifugation, Filtration, Anbindung an Beads, Amplifikation, insbesondere Polymerasekettenreaktion, durchgeführt, weil somit standardisierte Verfahren zur Ergebnisfindung eingesetzt werden und somit die Fehlerquote so niedrig wie möglich gehalten werden kann.

Die Analyse der fetalen Nukleinsäuren kann ein Verfahren ausgewählt aus einer Gruppe enthaltend Sequenzierung, Amplifikation, in-situ-Hybridisierung, insbesondere Fluoreszenz in-situ Hybridisierung, umfassen, wodurch rasch ein aussagekräftiges Ergebnis vorliegt und noch rechtzeitig vor dem Embryotransfer über das weitere Schicksal des Embryos entschieden werden kann.

Von Vorteil erweist sich, dass Erkrankungen, insbesondere monogenetische Erkrankungen, Blutgruppen, Geschlecht, Aneuploidien, HLA-Typen detektiert werden können und somit bereits vor Beginn der eigentlichen Schwangerschaft Gewissheit über die ursprüngliche Fragestellung vorliegt und nicht erst im Zuge der Schwangerschaft nach langem Zuwarten oder durch Gefährdung des Embryos ein Ergebnis erzielt wird.

Bei einem Verfahren zum Nachweis fetaler Nukleinsäuren aus der, den Embryo umgebenden Flüssigkeit innerhalb der Zona Pellucida, dem Blastocoel oder der Blastozystenhöhle umfassend die Schritte: a) Entnahme der zellfreien, fetale Nukleinsäure enthaltenden Flüssigkeit aus der, den Embryo umgebenden Flüssigkeit innerhalb der Zona Pellucida, dem Blastocoel oder der Blastozystenhöhle der Blastula oder Blastozyste, b) Anreicherung der, den Embryo umgebenden Flüssigkeit innerhalb der Zona Pellucida, im Blastocoel oder der Blastozystenhöhle enthaltenen fetalen Nukleinsäuren und c) Durchführung einer Analyse der im zellfreien Überstand vorhandenen Nukleinsäuren, wird eine Flüssigkeitsmenge ausgewählt aus einem Bereich mit einer unteren Grenze von 0,004 pl und einer oberen Grenze von 0,1 nl, insbesondere 0,04 nl, aus der den Embryo umgebenden Flüssigkeit innerhalb der Zona Pellucida, aus der im Blastocoel oder der Blastozystenhöhle der Blastula oder Blastozyste enthaltenen Flüssigkeit entnommen wird und in ein Flüssigkeitsvolumen, vorzugsweise bestehend oder beinhaltend das Kulturmedium, von mindestens 2 µl überführt und anschließend analysiert wird, um einerseits den Embryo, die Blastula bzw. Blastozyste nicht zu gefährden und andererseits aber ausreichend Material für die weitere Analyse zur Verfügung zu haben.

Die Isolation und Anreicherung der Nukleinsäure erfolgt durch Amplifikation, insbesondere Polymerasekettenreaktion, weil dadurch rasch eine ausreichende Menge an fetaler Nukleinsäure vorliegt um sie einer weiteren Analyse zugeführt werden kann.

Die Vorteile zur Isolierung und Analyse der Nukleinsäuren mittels standardisierten Verfahren entsprechen auch bei dieser alternativen Ausführungsform des Verfahrens, wo die zellfreien Nukleinsäuren aus der, den Embryo umgebenden Flüssigkeit innerhalb der Zona Pellucida, dem Blastocoel bzw. der Blastozystenhöhle gewonnen werden, jenen vorab beschriebenen. Zudem können mit dem alternativen erfindungsgemäßen Verfahren dieselben Parameter wie vorab beschrieben nachgewiesen und bestimmt werden.

Zum besseren Verständnis der Erfindung wird diese anhand der nachfolgenden Figur näher erläutert.

Es zeigt in stark schematisch vereinfachter Darstellung:

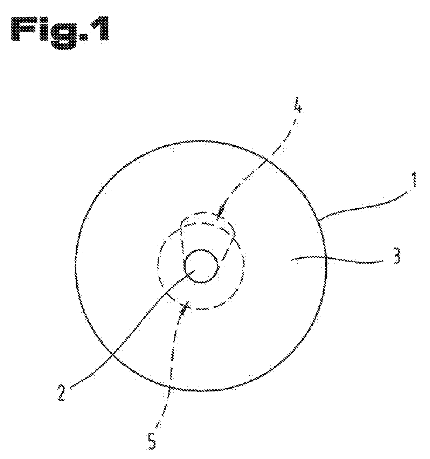

- Fig. 1: ein Kulturgefäß mit einer befruchteten Eizelle und den umgebenden Bereich.

Einführend sei festgehalten, dass in den unterschiedlich beschriebenen Ausführungsformen gleiche Teile mit gleichen Bezugszeichen versehen werden, wobei die in der gesamten Beschreibung enthaltenen Offenbarungen sinngemäß auf gleiche Teile mit gleichen Bezugszeichen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

Die vorliegende Erfindung beschreibt ein Verfahren, mit welchem Präimplantationsdiagnostik ohne Verwendung zellulären Materials durchgeführt werden kann.

Unter Embryo wird im Sinne der Erfindung jener zelluläre Teil der in-vitro Kultur verstanden, aus dem sich der eigentliche Embryo entwickelt inklusive die frühen Entwicklungsstadien, wie Morula, Blastula, Gastrula bzw. Blastozyste.

Die befruchtete Eizelle steht im Sinne der Erfindung für die unterschiedlichen frühen Entwicklungsstadien, wie Morula, Blastula, Gastrula bzw. Blastozyste, der Embryogenese.

Zur Durchführung des erfindungsgemäßen Verfahrens zum Nachweis fetaler Nukleinsäuren aus dem Überstand des Kulturmediums fetaler Zellkulturen einer in-vitro Fertilisationszellkultur wird zunächst ein Aliquot des Kulturmediumüberstands bzw. der gesamte Kulturmediumüberstand aus dem zur Kultivierung der befruchteten Eizelle verwendeten Kulturgefäß entnommen, der zellfrei ist und fetale Nukleinsäuren enthält. Anschließend wird das Kulturmedium mit der darin enthaltenen Nukleinsäure weiter verarbeitet und die im Überstand enthaltene fetale Nukleinsäuren isoliert. Diese isolierte Nukleinsäure des zellfreien Überstands wird nun einer weiteren Analyse unterzogen.

Als Kulturmedium für die befruchtete Eizelle bzw. den Embryo werden standardisierte Medien, wie HSA- bzw. rHSA-Medien verwendet, wodurch keine Fremd-DNA in die Zellkulturen eingebracht wird.

Bevor allerdings die befruchtete Eizelle kultiviert werden kann wird zuerst die Eizellentnahme (Follikelpunktion) mit anschließender Befruchtung der Eizelle mit der Samenzelle mittels IVF, ICSI, IMSI durchgeführt. Die so befruchteten Eizellen werden in ein Kulturmedium, auch Nährmedium genannt, gegeben. In einem Brutschrank werden diese nun inkubiert. Nach 16 bis 18 Stunden wird eine erste Kontrolle unter dem Mikroskop vorgenommen, um festzustellen, wie viele Eizellen sich mit den Samenzellen befruchten ließen. Es ist inzwischen möglich, befruchtete Eizellen bzw. Embryonen länger in Kultur zu halten um anschließend am Tag 5 (selten am Tag 6) nach der Follikelpunktion ein oder zwei Embryonen im Blastozysten-Stadium, bevorzugt expandiertem Blastozysten-Stadium, zu transferieren.

Dies bedeutet, dass aus der Gesamtheit aller über 3 bis 6 Tage kultivierten Embryonen, die am weitesten entwickelten und sowohl morphologisch als auch entsprechend dem erfindungsgemäßen Verfahren molekulargenetisch am unauffälligsten ausgewählt und transferiert werden.

Überraschenderweise ist bereits in der, den Embryo umgebenden Flüssigkeit innerhalb der Zona Pellucida, im Blastocoel bzw. der Blastozystenhöhle als auch im Kulturmediumüberstand eine ausreichende Menge extrazellulärer fetaler Nukleinsäuren vorhanden um sie einer weiter führenden Analyse unterziehen zu können. Es wird angenommen, dass die DNA aus apoptotischen Zellen innerhalb des Embryos kommt. Die DNA aus diesen Zellen wird freigesetzt und kann durch die Zona Pellucida durchtreten. Um eine molekulargenetische Analyse des zum Transfer vorgesehenen Embryos durchzuführen, ist daher die Entnahme zellulären Materials durch das erfindungsgemäße Verfahren obsolet. Es brauchen also Zellen keiner Art, also weder des späteren Embryos noch der ihn umgebenden Zellen bzw. des Morula-, Blastula-, Gastrula- oder Blastozystenstadiums entnommen werden. Da sich der zu entwickelnde Embryo in einem sehr aktiven Stadium im Gegensatz zur maternalen Eizelle und der paternalen Samenzelle befindet, ist der Anteil fetaler Nukleinsäuren im zellfreien Kulturmedium deutlich höher als jener der Eltern.

Durch die zellfreie Gewinnung der Nukleinsäuren aus dem Überstand ist deren weitere Verarbeitung, um eine Analyse durchführen zu können, deutlich erleichtert, weil keine zellulären Bestandteile abgetrennt werden müssen und somit womöglich fetale Nukleinsäuren verloren gehen würden, bzw. keine zellulären Verunreinigungen vorliegen, die eine Analyse womöglich stören würden.

Die fetale Nukleinsäure kann sowohl DNA als auch RNA sein, wobei meist Nukleinsäurefragmente vorliegen. So können frei zirkulierende DNA-Fragmente geringer Molekulargröße analysiert werden. Aber auch RNA, wie z.B. miRNA, siRNA oder mRNA, die beispielsweise für die weitere Analyse zunächst revers transkribiert wird, kann isoliert und analysiert werden.

Der zu analysierende Überstand des Kulturmediums kann aus mono oder sequentiellen Kulturmedien analysiert werden. Bei Einsatz von sequenziellen Kulturmedien wird im Zuge der Kultivierung der befruchteten Eizelle das Kulturmedium gegebenenfalls mehrmals gewechselt, wobei bei jedem Kulturmediumswechsel aus dem abgenommenen Kulturmedium die fetalen Nukleinsäuren isoliert und analysiert werden können. Mehrfacher Wechsel des Kulturmediums ist jedoch auch bei mono Kulturmedien möglich.

Der zellfreie, die Nukleinsäure enthaltende Überstand wird an zumindest einem der Tage 1 bis 6 der in-vitro Kultur entnommen und analysiert. Vorzugsweise wird der Überstand zwischen Tag 3 und 5 der in-vitro Kultur entnommen (ohne oder nach mechanischer, chemischer oder per Laser Eröffnung der Zona Pellucida), weil in diesem Zeitraum ausreichend fetale Nukleinsäuren im Überstand enthalten sind und dennoch das Ergebnis der Präimplantationsdiagnostik zeitgerecht vor dem Transfer des Embryos in den Uterus der Mutter vorliegt. Die Eröffnung der Zona Pellucida kann unmittelbar vor Entnahme des Überstands des Kulturmediums oder auch bereits bis zu 3 Tage vorher erfolgen.

Wird das Kulturmedium nicht gewechselt bzw. wird fetale Nukleinsäure vor einem Kulturmediumswechsel analysiert, so wird der Zellkulturüberstand aus einem die befruchtete Eizelle umgebenden Bereich vom zumindest 2-fachen Durchmesser der kultivierten befruchteten Eizelle entnommen. In Fig. 1 ist der Bereich, aus dem das Kulturmedium entnommen werden kann, nicht maßstäblich dargestellt. Fig. 1 zeigt ein Kulturgefäß 1 in dem die befruchtete Eizelle 2 im Kulturmedium 3 kultiviert wird. Ein möglicher die befruchtete Eizelle 2 umgebender Bereich 4 grenzt an die befruchtete Eizelle 2 an und erstreckt sich am oberen Rand derselben. Eine weitere Möglichkeit für den Bereich 5 ist ebenfalls in Fig. 1 dargestellt, wobei sich dieser radial um die Eizelle 2 erstreckt. Um eine ausreichende Menge an fetaler Nukleinsäure zu gewinnen, wird vorzugsweise das Volumen aus dem Abstand des doppelten maximalen Durchmessers der befruchteten Eizelle 2 entnommen.

Es werden zumindest 2 µl des Zellkulturüberstands entnommen um sicher zu stellen, dass zumindest ein paar Moleküle fetaler Nukleinsäure enthalten sind, die zwar in der Regel ohnehin amplifiziert werden bevor sie der weiteren Analyse unterzogen wird.

Allerdings kann bei einem Kulturmediumwechsel bzw. zum Zeitpunkt des Embryotransfers in den Uterus auch das gesamte Kulturmedium der weiteren Analyse unterzogen werden.

Die Isolierung der fetalen Nukleinsäuren aus dem zellfreien Zellkulturüberstand erfolgt durch aus dem Stand der Technik bekannte Methoden, die zur Anreicherung von Nukleinsäuren dienen. Dies sind insbesondere Verfahren ausgewählt aus einer Gruppe umfassend Zentrifugation, Filtration, Anbindung an Beads, Amplifikation, insbesondere Polymerasekettenreaktion, etc.

Allerdings kann auch ohne Anreicherung oder Isolierung die Anwesenheit und Menge fetaler DNA beispielsweise durch spektrophotometrische Methoden nachgewiesen werden. Beispielsweise kann hiermit bereits aus einer kleinen Probenmenge (1 µl) und dem daraus erhaltenen Spektrum die Menge der in der Probe vorhandenen Nukleinsäuren (als DNA oder RNA) berechnen werden. Daraus können Hinweise für die weitere Entwicklung der befruchteten Eizelle bzw. des Embryos gewonnen werden.

Die Analyse der fetalen Nukleinsäuren erfolgt ebenfalls mit aus dem Stand der Technik bekannten Verfahren, wobei vorzugsweise ein Verfahren ausgewählt aus einer Gruppe umfassend eine Sequenzierung, Amplifikation, in-situ Hybridisierung, insbesondere Fluoreszenz in-situ Hybridisierung, durchgeführt werden. Die Untersuchung der Nukleinsäuren erfolgt allerdings je nach Fragestellung mittels unterschiedlicher Analyseverfahren und dauert gewöhnlich zwischen zwei und 24 Stunden. Beispielsweise kann die Amplifikation und gegebenenfalls Isolierung der Nukleinsäure mittels Amplifikationsmethoden erfolgen, womit Mengen eines Zelläquivalents DNA mittels whole genome amplification nachgewiesen werden können. Der FisH(Fluoreszenz in-situ Hybridisierung)-Test beispielsweise testet auf Chromosomenaberrationen, sehr schwerwiegende Veränderungen des Genoms. Einzelne Gene werden dann untersucht, wenn bei den Eltern eine Disposition zu einem Gendefekt vorliegt, wenn also eine bestimmte Erbkrankheit in der Familie gehäuft vorkommt. Neben den am häufigsten verwendeten Methoden der PCR-Amplifikation und der Fluoreszenz in-situ Hybridisierung stehen auch noch die aus dem Stand der Technik bekannten Methoden der "whole gene amplification" für die Präimplantationsdiagnostik zur Verfügung. Auch "Karyomapping" kann als mögliches Analyseverfahren für die mit dem erfindungsgemäßen Verfahren gewonnen Nukleinsäuren durchgeführt werden.

Durch die Analyse zellfreier genomischer bzw. mitochondrialer DNA eines in Kultur befindlichen Embryos können wichtige Parameter für die Erfolgsaussichten der Implantation, die Lebensfähigkeit und die genetische Prädisposition des sich daraus entwickelnden Lebewesens gewonnen werden. Es können Erkrankungen, insbesondere monogenetische Erkrankungen, Blutgruppen, Geschlecht, Aneuploidien, HLA-Typen, etc. detektiert werden. Aber selbstverständlich können auch andere Parameter auf molekulargenetischer Basis bestimmt werden. Mit dem erfindungsgemäßen Verfahren können auch biologische Marker detektiert werden, die mit chromosomalen Aneuploidien in Zusammenhang stehen und auf molekulargenetischer Ursache basieren.

In einer alternativen Ausführungsvariante der Erfindung kann auch ein Verfahren zum Nachweis fetaler Nukleinsäuren aus der, den Embryo umgebenden Flüssigkeit innerhalb der Zona Pellucida, dem Blastocoel oder der Blastozystenhöhle verwendet werden, um Präimplantationsdiagnostik zu betreiben ohne zelluläres Material zu verwenden. In einem ersten Schritt wird zellfreie, fetale Nukleinsäure enthaltende Flüssigkeit aus der, den Embryo umgebenden Flüssigkeit innerhalb der Zona Pellucida, dem Blastocoel oder der Blastozystenhöhle der Blastula oder Blastozyste entnommen. Nachfolgend wird die in der, den Embryo umgebenden Flüssigkeit innerhalb der Zona Pellucida, im Blastocoel oder der Blastozystenhöhle enthaltene fetale Nukleinsäuren angereichert bzw. isoliert. Zuletzt wird eine molekularbiologische Analyse der im zellfreien Überstand vorhandenen Nukleinsäuren durchgeführt.

Beispielsweise kann die Zona Pellucida am Tag 3 geöffnet werden und das Kulturmedium an den Tagen 3 bis 5 gesammelt werden. Alternativ kann am Tag 3 der innere Teil auch gespült werden und das Medium gesammelt werden.

Aus dem Blastocoel oder der Blastozystenhöhle können am Tag 5 Proben gewonnen werden.

Aus der, den Embryo umgebenden Flüssigkeit innerhalb der Zona Pellucida, dem Blastocoel oder der Blastozystenhöhle werden zumindest 0,004 pl, insbesondere 0,004 nl bis 0,04 nl, Flüssigkeit entnommen. Vorzugsweise werden aus der den Embryo umgebenden Flüssigkeit innerhalb der Zona Pellucida zumindest 0,004 nl entnommen und aus dem Blastocoel oder der Blastozystenhöhle der Blastula oder Blastozyste zumindest 0,04 pl bis 0,04 nl der Flüssigkeit entnommen und in ein Flüssigkeitsvolumen, idealerweise bestehend oder beinhaltend das Kulturmedium, von mindestens 2 µl überführt und anschließend analysiert, um jedenfalls Moleküle fetaler Nukleinsäuren in der entnommenen Flüssigkeit des Blastocoels bzw. der Blastozystenhöhle bzw. der den Embryo umgebenden Flüssigkeitsbereich innerhalb der Zona Pellucida, enthalten zu haben.

Die in der Flüssigkeit des Blastocoels bzw. der Blastozystenhöhle enthaltene Nukleinsäure wird vorzugsweise durch Amplifikation, insbesondere Polymerasekettenreaktion, angereichert.

Es können die vorab beschriebenen Isolations- und Analyseverfahren verwendet werden, sowie dieselben Parameter bestimmt werden.

Die Ausführungsvarianten des Verfahrens zum Nachweis fetaler Nukleinsäuren aus dem Überstand des Kulturmediums fetaler Zellkulturen einer in-vitro Fertilisation sind nicht auf die speziell dargestellten Ausführungsvarianten desselben eingeschränkt, sondern vielmehr sind auch diverse Kombinationen der einzelnen Ausführungsvarianten untereinander möglich und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mit umfasst.

Der Ordnung halber sei abschließend darauf hingewiesen, dass zum besseren Verständnis die Bestandteile der Fig. 1 teilweise unmaßstäblich und/oder vergrößert und/oder verkleinert dargestellt wurden.

Die den eigenständigen erfinderischen Lösungen zugrundeliegende Aufgabe kann der Beschreibung entnommen werden.

### Bezugszeichenaufstellung

- 1: Kulturgefäß
- 2: Eizelle
- 3: Kulturmedium
- 4: Bereich
- 5: Bereich

## Patentansprüche

1. Verfahren zum Nachweis fetaler Nukleinsäuren aus dem Überstand des Kulturmediums fetaler Zellkulturen einer in-vitro Fertilisation umfassend die Schritte: a) Entnahme des zellfreien, fetale Nukleinsäure enthaltenden Überstands des in-vitro Fertilisationskulturmediums aus dem zur Kultivierung der befruchteten Eizelle verwendeten Kulturgefäß, b) Isolierung der im Überstand enthaltenen fetalen Nukleinsäuren und c) Durchführung einer Analyse der im zellfreien Überstand vorhandenen Nukleinsäuren.

2. Verfahren nach Anspruch 1, **dadurch gekennzeichnet, dass** als fetale Nukleinsäure DNA und/oder RNA analysiert wird.

3. Verfahren nach einem der Ansprüche 1 oder 2, **dadurch gekennzeichnet, dass** der Zellkulturüberstand mono und/ oder sequentieller Kulturmedien analysiert wird.

4. Verfahren nach einem der Ansprüche 1 bis 3, **dadurch gekennzeichnet, dass** der zellfreie, die Nukleinsäure enthaltende Überstand an zumindest einem der Tage 1 bis 6 der in-vitro Kultur analysiert wird.

5. Verfahren nach einem der Ansprüche 1 bis 4, **dadurch gekennzeichnet, dass** der Überstand des Kulturmediums (3) des Kulturgefäßes (1) aus einem die befruchtete Eizelle (2) umgebenden Bereich (4, 5) vom zumindest zweifachen Durchmesser der kultivierten befruchteten Eizelle (2) entnommen wird.

6. Verfahren nach einem der Ansprüche 1 bis 5, **dadurch gekennzeichnet, dass** zumindest 2 µl des Zellkulturüberstands analysiert werden.

7. Verfahren nach einem der Ansprüche 1 bis 6, **dadurch gekennzeichnet, dass** die Eizellhülle (Zona Pellucida) der befruchteten Eizelle mechanisch, chemisch oder per Laser 1 bis 6 Tage vor Entnahme des Zellkulturüberstandes geöffnet wird.

8. Verfahren nach einem der Ansprüche 1 bis 7, **dadurch gekennzeichnet, dass** die Isolierung der fetalen Nukleinsäuren durch Anreicherung, insbesondere einem Verfahren ausgewählt aus einer Gruppe umfassend Zentrifugation, Filtration, Anbindung an Beads, Amplifikation, insbesondere Polymerasekettenreaktion, durchgeführt wird.

9. Verfahren nach einem der Ansprüche 1 bis 8, **dadurch gekennzeichnet, dass** die Analyse der fetalen Nukleinsäuren ein Verfahren ausgewählt aus einer Gruppe umfassend eine Sequenzierung, Amplifikation, in-situ Hybridisierung, insbesondere Fluoreszenz in-situ Hybridisierung, umfasst.

10. Verfahren nach einem der Ansprüche 1 bis 9, **dadurch gekennzeichnet, dass** Erkrankungen, insbesondere monogenetische Erkrankungen, Blutgruppen, Geschlecht, Aneuploidien, HLA-Typen des Embryos detektiert werden.

11. In vitro Verfahren zum Nachweis fetaler Nukleinsäuren aus der, den Embryo umgebenden Flüssigkeit innerhalb der Eizellhülle (Zona Pellucida), aus dem Blastocoel oder aus der Blastozystenhöhle umfassend die Schritte: a) Entnahme der zellfreien, fetale Nukleinsäure enthaltenden Flüssigkeit aus der, den Embryo umgebenden Flüssigkeit innerhalb der Eizellhülle (Zona Pellucida), dem Blastocoel oder der Blastozystenhöhle der Blastula oder Blastozyste, b) Anreicherung der, in jener den Embryo umgebenden Flüssigkeit innerhalb der Eizellhülle (Zona Pellucida), im Blastocoel oder der Blastozystenhöhle enthaltenen fetalen Nukleinsäuren und c) Durchführung einer Analyse der im zellfreien Überstand vorhandenen Nukleinsäuren.

12. Verfahren nach Anspruch 11, **dadurch kennzeichnet, dass** eine Flüssigkeitsmenge ausgewählt aus einem Bereich mit einer unteren Grenze von 0,004 pl, insbesondere von 0,004 nl und einer oberen Grenze von 0,1 nl aus der den Embryo umgebenden Flüssigkeit innerhalb der Eizellhülle (Zona Pellucida), aus der im Blastocoel oder der Blastozystenhöhle der Blastula oder Blastozyste enthaltenen Flüssigkeit entnommen wird und in ein Flüssigkeitsvolumen, vorzugsweise bestehend oder beinhaltend das Kulturmedium, von mindestens 2 µl überführt und anschließend analysiert wird.

13. Verfahren nach Anspruch 11 oder 12, **dadurch kennzeichnet, dass** die Anreicherung der Nukleinsäure durch Amplifikation, insbesondere Polymerasekettenreaktion erfolgt.

14. Verfahren nach einem der Ansprüche 11 bis 13, **dadurch gekennzeichnet, dass** die Isolierung der fetalen Nukleinsäuren durch Anreicherung, insbesondere einem Verfahren ausgewählt aus einer Gruppe umfassend Zentrifugation, Filtration, Anbindung an Beads, Amplifikation, insbesondere Polymerasekettenreaktion, durchgeführt wird.

15. Verfahren nach einem der Ansprüche 11 bis 14, **dadurch gekennzeichnet, dass** die Analyse der fetalen Nukleinsäuren ein Verfahren ausgewählt aus einer Gruppe umfassend eine Sequenzierung, Amplifikation, in-situ Hybridisierung, insbesondere Fluoreszenz in-situ Hybridisierung, umfasst.

16. Verfahren nach einem der Ansprüche 11 bis 15, **dadurch gekennzeichnet, dass** Erkrankungen, insbesondere monogenetische Erkrankungen, Blutgruppen, Geschlecht, Aneuploidien, HLA-Typen des Embryos detektiert werden.

## Claims

1. A method for analyzing fetal nucleic acids from the supernatant of the culture medium of fetal cell cultures of in vitro fertilization, comprising the steps: a) extracting cell-free supernatant containing fetal nucleic acid from the in vitro fertilization culture medium from the culture vessel used for cultivating the fertilized egg cell, b) isolating the fetal nucleic acids contained in the supernatant and c) performing an analysis of the nucleic acids present in the cell-free supernatant.

2. The method according to claim 1, **characterized in that** DNA and/or RNA are/is analyzed as fetal nucleic acid.

3. The method according to any one of claims 1 or 2, **characterized in that** the cell culture supernatant of mono and/or sequential culture media is analyzed.

4. The method according to any one of claims 1 to 3, **characterized in that** the cell-free supernatant containing the nucleic acid is analyzed on at least one of days 1 to 6 of the in vitro culture.

5. The method according to any one of claims 1 to 4, **characterized in that** the supernatant of the culture medium (3) of the culture vessel (1) is extracted from an area (4, 5) surrounding the fertilized egg cell (2), which area has at least twice the diameter of the cultivated fertilized egg cell (2).

6. The method according to any one of claims 1 to 5, **characterized in that** at least 2 µl of the cell culture supernatant is analyzed.

7. The method according to any one of claims 1 to 6, **characterized in that** the egg cell membrane (zona pellucida) of the fertilized egg cell is opened mechanically, chemically or by laser 1 to 6 days prior to the extraction of the cell culture supernatant.

8. The method according to any one of claims 1 to 7, **characterized in that** the isolation of the fetal nucleic acids is performed by enrichment, in particular by a method selected from a group comprising centrifugation, filtration, binding to beads and amplification, in particular a polymer chain reaction.

9. The method according to any one of claims 1 to 8, **characterized in that** the analysis of the fetal nucleic acids comprises a method selected from a group comprising sequencing, amplification and in situ hybridization, in particular fluorescence in situ hybridization.

10. The method according to any one of claims 1 to 9, **characterized in that** diseases, in particular monogenetic diseases, blood groups, the sex, aneuploidies and HLA types of the embryo are detected.

11. An in vitro method for analyzing fetal nucleic acids from the fluid surrounding the embryo within the egg cell membrane (zona pellucida), from the blastocoel or from the blastocyst cavity, comprising the steps: a) extracting the cell-free fluid containing fetal nucleic acid from the fluid surrounding the embryo within the egg cell membrane (zona pellucida), the blastocoel or the blastocyst cavity of the blastula or blastocyst, b) enriching the fetal nucleic acids contained in that fluid surrounding the embryo within the egg cell membrane (zona pellucida), in the blastocoel or the blastocyst cavity and c) performing an analysis of the nucleic acids present in the cell-free supernatant.

12. The method according to claim 11, wherein a quantity of fluid selected from a range with a lower limit of 0.004 pl, in particular 0.004 nl, and an upper limit of 0.1 nl is extracted from the fluid surrounding the embryo within the egg cell membrane (zona pellucida), from the fluid contained in the blastocoel or blastocyst cavity of the blastula or blastocyst and transferred into a fluid volume, preferably consisting of or containing the culture medium, of at least 2 µl and then analyzed.

13. The method according to claim 11 or 12, wherein the enrichment of the nucleic acid is performed by amplification, in particular a polymer chain reaction.

14. The method according to any one of claims 11 to 13, **characterized in that** the isolation of the fetal nucleic acids is performed by enrichment, in particular by a method selected from a group comprising centrifugation, filtration, binding to beads and amplification, in particular a polymerase chain reaction.

15. The method according to any one of claims 11 to 14, **characterized in that** the analysis of the fetal nucleic acids comprises a method selected from a group comprising sequencing, amplification and in situ hybridization, in particular fluorescence in situ hybridization.

16. The method according to any one of claims 11 to 15, **characterized in that** diseases, in particular monogenetic diseases, blood groups, the sex, aneuploidies and HLA types of the embryo are detected.

## Revendications

1. Procédé de détection d'acides nucléiques foetaux à partir du surnageant du milieu de culture de cultures cellulaires foetales d'une fertilisation in vitro comprenant les étapes suivantes : a) l'extraction du surnageant sans cellules contenant l'acide nucléique foetal du milieu de culture de fertilisation in vitro à partir du récipient de culture utilisé pour la culture des ovules fertilisés, b) l'isolation des acides nucléiques foetaux contenus dans le surnageant et c) la réalisation d'une analyse des acides nucléiques présents dans le surnageant sans cellules.

2. Procédé selon la revendication 1, **caractérisé en ce qu'**un ADN et/ou un ARN est analysé en tant qu'acide nucléique foetal.

3. Procédé selon l'une quelconque des revendications 1 ou 2, **caractérisé en ce que** le surnageant de culture cellulaire de milieux de culture mono et/ou séquentiels est analysé.

4. Procédé selon l'une quelconque des revendications 1 à 3, **caractérisé en ce que** le surnageant sans cellules contenant l'acide nucléique est analysé à au moins un des jours 1 à 6 de la culture in vitro.

5. Procédé selon l'une quelconque des revendications 1 à 4, **caractérisé en ce que** le surnageant du milieu de culture (3) du récipient de culture (1) est extrait d'une zone (4, 5) entourant les ovules fertilisés (2) d'un diamètre au moins double des ovules fertilisés cultivés (2).

6. Procédé selon l'une quelconque des revendications 1 à 5, **caractérisé en ce qu'**au moins 2 µl du surnageant de culture cellulaire sont analysés.

7. Procédé selon l'une quelconque des revendications 1 à 6, **caractérisé en ce que** l'enveloppe (Zona Pellucida) des ovules fertilisés est ouverte mécaniquement, chimiquement ou par laser 1 à 6 jours avant l'extraction du surnageant de culture cellulaire.

8. Procédé selon l'une quelconque des revendications 1 à 7, **caractérisé en ce que** l'isolation des acides nucléiques foetaux est réalisée par enrichissement, notamment par un procédé choisi dans le groupe constitué par la centrifugation, la filtration, la liaison à des billes, l'amplification, notamment la réaction en chaîne par polymérase.

9. Procédé selon l'une quelconque des revendications 1 à 8, **caractérisé en ce que** l'analyse des acides nucléiques foetaux comprend un procédé choisi dans le groupe comprenant un séquençage, une amplification, une hybridation in situ, notamment une hybridation in situ par fluorescence.

10. Procédé selon l'une quelconque des revendications 1 à 9, **caractérisé en ce que** des maladies, notamment des maladies monogénétiques, des groupes sanguins, le sexe, des aneuploïdies, des types HLA de l'embryon sont détectées.

11. Procédé in vitro pour la détection d'acides nucléiques foetaux à partir du liquide entourant l'embryon dans l'enveloppe de l'ovule (Zona Pellucida), à partir du blastocèle ou à partir de la cavité du blastocyste, comprenant les étapes suivantes : a) l'extraction du liquide sans cellules contenant l'acide nucléique foetal à partir du liquide entourant l'embryon dans l'enveloppe de l'ovule (Zona Pellucida), à partir du blastocèle ou à partir de la cavité du blastocyste du blastula ou du blastocyste, b) l'enrichissement des acides nucléiques foetaux contenus dans le liquide entourant l'embryon dans l'enveloppe de l'ovule (Zona Pellucida), dans le blastocèle ou la cavité du blastocyste, et c) la réalisation d'une analyse des acides nucléiques présents dans le surnageant sans cellules.

12. Procédé selon la revendication 11, **caractérisé en ce qu'**une quantité de liquide choisie dans une plage ayant une limite inférieure de 0,004 pl, notamment de 0,004 nl et une limite supérieure de 0,1 ni est soutirée à partir du liquide entourant l'embryon dans l'enveloppe de l'ovule (Zona Pellucida), à partir du liquide contenu dans le blastocèle ou la cavité du blastocyste du blastula ou du blastocyste, et transférée dans un volume de liquide, de préférence constitué par ou contenant le milieu de culture, d'au moins 2 µl, puis analysée.

13. Procédé selon la revendication 11 ou 12, **caractérisé en ce que** l'enrichissement de l'acide nucléique a lieu par amplification, notamment par réaction en chaîne par polymérase.

14. Procédé selon l'une quelconque des revendications 11 à 13, **caractérisé en ce que** l'isolation des acides nucléiques foetaux est réalisée par enrichissement, notamment par un procédé choisi dans le groupe comprenant la centrifugation, la filtration, la liaison à des billes, l'amplification, notamment la réaction en chaîne par polymérase.

15. Procédé selon l'une quelconque des revendications 11 à 14, **caractérisé en ce que** l'analyse des acides nucléiques foetaux comprend un procédé choisi dans un groupe comprenant un séquençage, une amplification, une hybridation in situ, notamment une hybridation in situ par fluorescence.

16. Procédé selon l'une quelconque des revendications 11 à 15, **caractérisé en ce que** des maladies, notamment des maladies monogénétiques, des groupes sanguins, le sexe, des aneuploïdies, des types HLA de l'embryon sont détectées.
